# EUROPEAN PATENT APPLICATION

(11) **EP 1 736 098 A1**
(43) Date of publication of application: **27.12.2006**
(21) Application number: 05730446.1
(22) Date of filing: 14.04.2005
(51) Int. Cl.: A61B 5/107, A61B 5/00, A61B 19/00

(54) **FACE IMAGING DEVICE**

(30) Priority: 15.04.2004 JP 2004120289; 12.07.2004 JP 2004205057
(71) Applicant: Moritex Corporation, Tokyo 150-0001 (JP)
(72) Inventor: ASAI, Yoshiyuki c/o Saitama Factory, Saitama-shi, Saitatama 3380837 (JP); SUEYOSHI, Naoji c/o Saitama Factory, Saitama-shi, Saitama 3380837 (JP)
(74) Representative: Stachow, Ernst-Walther
(86) International application number: PCT/JP2005/007204
(87) International publication number: WO 2005/099575

(57) **Abstract**

A face imaging device capable of imaging a face from plural directions under the same imaging condition, capable of illuminating the face at uniform brightness and capable of obtaining a clear image without causing halation in the image. A face imaging device has an illumination light source (L) provided in a housing (2) in the front of which an opening (6) for inserting a face (3) as the imaging object is formed; a light diffusion plate (7) for diffusing illumination light to illuminate the front side of the face (3) and provided between the opening and the illumination light source (L); an imaging camera (4) provided behind the light diffusion plate (7); a head section supporting member (9) whose position is adjustable in forward-backward and left-right directions and against which a head top side non-observation region if the face (3) is pressed to restrict the position of a head section; a chin placement base (10) for restricting the position of the chin depending on an imaging direction such that the front of the face (3) and left and right cheeks face straight toward the imaging camera (4); and a light shielding body (15) between the illumination light (L) and the imaging camera (4).

## Description

### Technical Field

The present invention concerns a face imaging device capable of imaging a face from plural directions under an identical imaging condition.

### Background Art

Laser treatment of irradiating a laser light to a skin surface and subcutaneous portions to be treated has been conducted, for example, in dermatology, plastic surgery, orthopedic surgery, etc.
Particularly, in a case where a portion to be treated is present on a face such as in the therapy for pimples, since patient can not directly observe the portion to be treated, a doctor puts an image taken by a digital camera into a computer and projects the same on a monitor for conducting informed consent before surgery operation or confirmation for the effect of treatment and explanation to the patient after surgery.

However, in a case of imaging the portion to be treated, since an image is taken at an optional distance, the size of the portion to be treated differs subtly, as well as the brightness of the picked-up image is also different depending on the brightness of the surroundings, the background color, the direction of light, as well as the brightness of an object in view of the characteristic of a digital camera.
Accordingly, while it was possible for conducting informed consent of explaining the method of treatment, etc. before surgery only based on the image picked-up before the surgery and explaining the effect of treatment only based on the image picked-up after the surgery, since the brightness, etc. are different between two images picked-up before and after the surgery, they can not be compared objectively and it was difficult to explain them appropriately.

In view of the above, in order to make it possible for imaging a face always at identical distance/identical brightness, the present applicant already has proposed a face imaging device, in which a light shielding box formed with an opening for inserting a face is incorporated with an imaging camera for imaging a face, and an illumination device for illuminating a UV-illumination light and a visible illumination light selectively to the opening, and a face cushion is disposed to the periphery of the opening which is abutted against the periphery of the face, for closing a gap between the opening and the face thereby preventing leakage of an external light.
Patent Document 1: JP-A No. 2004-81735

However, since the face can be imaged only from the front by the means, while skins in a T-shaped zone from a forehead to a nose and the front of cheeks can be taken, the direction of the face can not be restricted upon imaging the surface on the lateral side of the cheek.

Further, in a case of illuminating an illumination light, while a circular fluorescent lamp or an LED has been used as an illumination light source, when the illumination light is illuminated directly to the face, this partially results in shining and the state of the skin can not be recognized accurately based on the picked-up images
Accordingly, it has been demanded for illumination at an entirely uniform brightness and the present inventors have experimentally prepared an imaging device in which a light diffusion plate is located between a face as an object and an illumination light source for diffusing the light illuminated from the illumination light source and illuminating the front side of the face.

While shining at the face skin was not caused, since the imaging camera and the illumination light source are situated on the identical side relative to the light diffusion plate, this resulted in an additional problem that a portion of a light illuminated from the illumination light source directly enters the imaging camera or a reflection light reflected at the back of the light diffusion plate enters the imaging camera thereby causing a weak halation to a portion of a screen making the thus image not clear.

### Disclosure of the Invention

### Subject to be Solved by the Invention

Then, the present invention has a technical subject of making it possible for imaging a face from plural directions under an identical imaging conditions, illuminating the face entirely at a uniform brightness, and obtaining a clear image without causing halation in the picked-up image.

### Means for Solving the Subject

For solving the subject, the present invention provides a face imaging device in which an illumination light source for illuminating an illumination light to a face is provided in a housing formed at the front thereof with an opening for inserting the face as an object, a light diffusion plate for diffusing illumination light illuminated from the illumination light source and illuminating the front side of the face is provided between the opening and the illumination light source, an imaging camera for imaging the face through a permeation hole formed in the light diffusion plate is provided at the back thereof in which a face holding mechanism is provided for holding the face as an object at a focused focal point of the imaging camera by a head support member adjustable for forward-to-backward and vertical positions by abutment against a non-observation region on head top side of the face inserted in the casing and a jaw-rest for regulating the position of a jaw in accordance with the imaging direction such that the front side of the face and the right and left cheeks oppose to the imaging camera,
and a light shielding body is disposed between the illumination light source and the imaging camera for shading, among light illuminated from the illumination light source, a light incident to the imaging camera not transmitting the permeation hole.

### Effect of the Invention

According to the present invention, a head and a jaw are supported in the casing, and the face is positioned to a focused focal point of the imaging camera.
In this case, since the head support member is put against a non-observation region on head top side such as a hair portion which is not necessary for image data when observing the skin, a necessary image is not concealed by the support member and the image of the necessary portion can be taken entirely.
Further, the jaw rest restricts the position of the jaw in accordance with the imaging direction such that the front side of the face and the light or left cheek portion is faced to the imaging camera.
Accordingly, since the jaw is rested on the jaw rest and supported at a position in accordance with the direction of the face and the forehead is supported by the head support member, the image of the face can be taken at an identical distance and at an identical angle relative to the imaging camera.
In this case, by making the imaging region smaller than the face, since it does not suffer from the effect of the background brightness, the image can be taken at an identical brightness.

Further, since the light illuminated from the illumination light source is diffused through the light diffusion plate and illuminates the entire front side of the face at a uniform brightness, it does not cause shining.
Further, since the light shielding body is located between the illumination light source and the imaging camera for shielding the light incident to the imaging camera not transmitting the permeation hole, a light incident directly to the imaging camera or a light reflected at the back of the light diffusion plate and incident to the imaging camera are cut off, among the light illuminated from the illumination light source, a clear image can be obtained with no halation.
In a case of using a reflection mirror as the light shielding body, since the light illuminated from the illumination light source to the imaging camera can be reflected on the side of the diffusion plate, the illuminated light can be effectively utilized with no loss as the illumination light.

Further, in a case of forming the protrusion to be abutted against the back of the lower jawbone to the jaw rest, the jaw can be positioned accurately.
Further, for restricting the position of the jaw in accordance with the imaging direction, the jaw rest may be adapted such that a plurality of jaw rest portions are arranged or a single jaw rest portion is disposed movably in accordance with the imaging direction.

In this case, the head support member that supports the non-observation region on head top side of the face at one point may be disposed in a manner capable of moving rightward and leftward in accordance with the imaging direction.
Further, in a case of supporting the non-observation region on head top side at two right and left points, since the face is supported at three points of the jaw and the head, the face can be supported extremely stably without jolting the head support member even when the direction of the face is changed.

### Best Mode for Carrying Out the Invention

In this embodiment, a subject of imaging a face from plural directions under an identical imaging condition can be attained by providing a face holding mechanism capable of holding a face simply to a focused focal point of an imaging camera while giving no hindrance to the imaging operation.

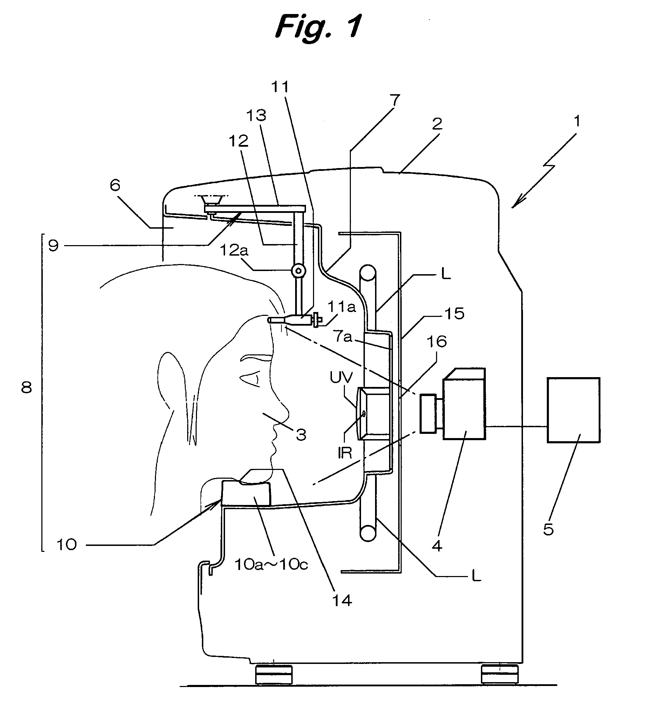

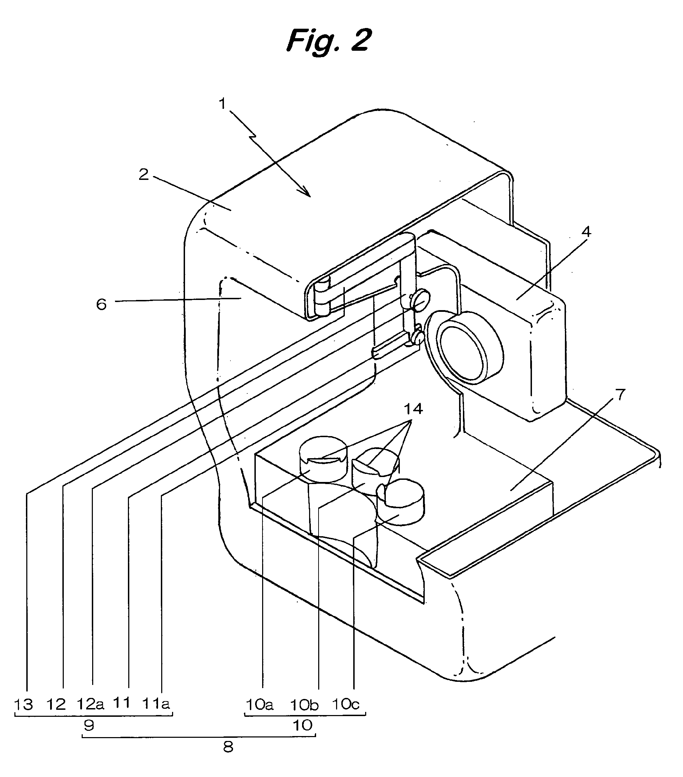

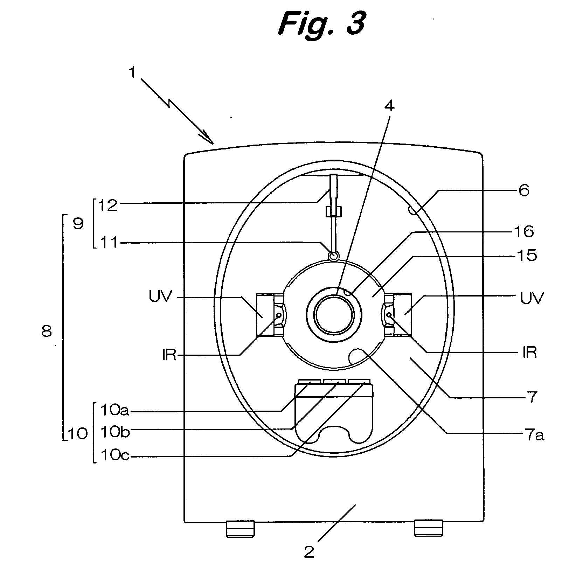

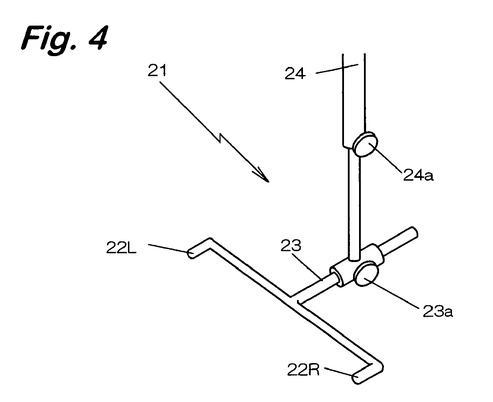

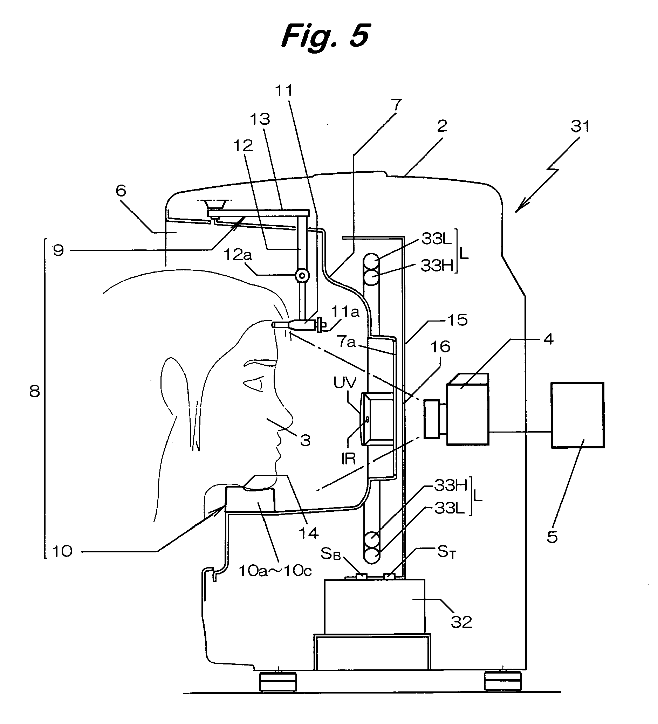

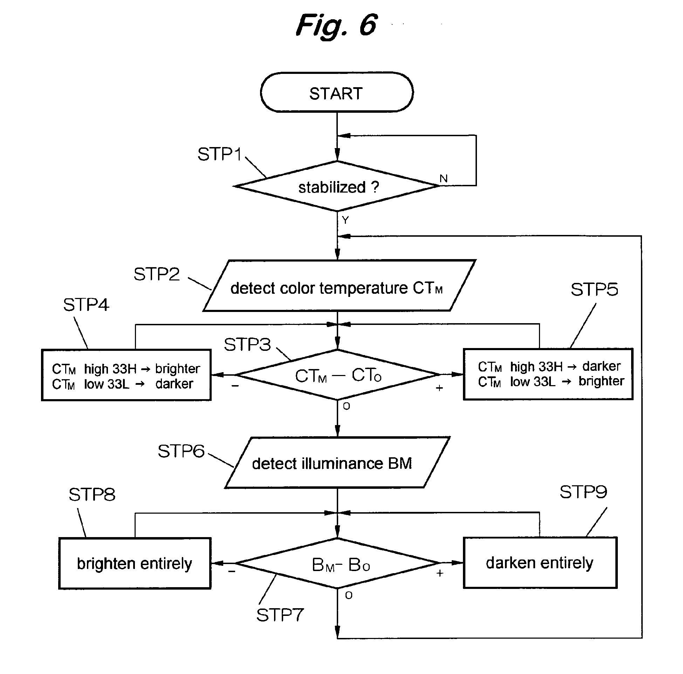

Fig. 1 is a cross sectional view of a face imaging device according to the present invention, Fig. 2 is a partially cut away perspective view while omitting an internal mechanism, Fig. 3 is a front elevational outer looking view, Fig. 4 is an explanatory view showing a principal portion of another embodiment, Fig. 5 is an explanatory view showing a further embodiment, and Fig. 6 is a flow chart showing an example of illuminance/color temperature control.

### Embodiment 1

A face imaging device 1 shown in Fig. 1 to Fig. 3 has an imaging camera 4 for imaging a face 3 provided in a casing 2 in which an image signal outputted from the imaging camera 4 can be taken into a computer 5 and projected.
An opening 6 for inserting the face 3 is formed at the front of the casing 2, an illumination light source L for illuminating a visible light to the face 3 is located to the inside thereof, an opaque light diffusion plate 7 is situated between the opening 6 and the illumination light source L for diffusing the light illuminated from the illumination light source L and illuminating the front side of the face 3, and an imaging camera 4 for imaging the face through a permeation hole 7a formed in the light diffusion plate 7 is disposed at the back thereof.
In this embodiment, a circular or straight tubular white light emission tube W is used as the illumination light source L, which is disposed so as to surround the optical axis of the imaging camera 4.

Then, a plate-like light shielding body 15 is located between the illumination light source L and the imaging camera 4 for shielding a light incident to the imaging camera 4 while not transmitting the permeation hole 7a of the light diffusion plate 7, among the light illuminated from the illumination light source L.
Thus, the light from the illumination light source L transmitting through the light diffusion plate 7, reflected at the face 3 and passing through the permeation hole 7a is incident to the imaging camera 4.
Further, the light shielding body 15 is formed as a reflection mirror that reflects a light emitted from the illumination light source L to the imaging camera 4 toward the light diffusion plate 7, and a permeation hole 16 is perforated at a portion crossing an imaging area of the imaging camera 4 in accordance with the view angle thereof such that the light shielding body 15 is not taken upon imaging.

Further, in this embodiment, a UV-light source UV such as a xenone lamp for directly illuminating a UV light to the face and an auxiliary light source IR such as an infrared LED for directly illuminating a focusing auxiliary illumination light of the imaging camera 4 are arranged outside of the imaging area of the imaging camera 4 at a position not in the shade of the light diffusion plate as viewed from the side of the opening 6.

In a case of imaging UV-light images, since a visible light illuminated from the illumination light source generates noises and the illumination light source L is put off, it may result in a worry that the imaging camera 4 does not operate for the sake of the insufficiency in the amount of a measuring light in a case of using a camera having an auto-focusing function.
Accordingly, the auxiliary light source IR comprising the infrared LED is lit to operate the auto-focusing only upon conducting focusing before UV imaging, and the auxiliary light source IR is put off upon imaging the UV-image after the completion of the focusing.
Thus, since only the reflection light of the UV-light can be taken as images for the UV-ray images, noises other than those of the UV-light can be restricted.
Further, the auxiliary light source IR is not limited only to the infrared LED but a white LED or a visible light bulb may also be used. Further, instead of lighting up the auxiliary light source IR, it may also be adapted such that the illumination light source L is lit up upon conducting the focusing before imaging the UV-ray images to operate the auto-focusing, and it may be put off upon imaging the UV images after completion of the focusing.

In UV-ray images, since the portion for "aged keratinoide" (not illustrated) appears white, the portion for "oily skin" appears orange, which can be distinguished easily but "normal skin", "dry skin", "melanin" appear purple respectively and can not be distinguished clearly.
Accordingly, image processing is applied by the computer 5 in which the brightness and the contrast are adjusted based on the difference between visible light images and UV-light images, to output synthesized images capable of distinguishing white "aged keratinoide", orange "oily skin" and bright "normal skin", "dry skin" of an intermediate tone, and dark "melanin".

In the opening 6, a face holding mechanism 8 for holding a face 3 as an object to be imaged is held at a focused focal point of the imaging camera 4.
The face holding mechanism 8 has a head support member 9 capable of adjusting the forward-to-backward and vertical positions for controlling the position of the head by abutment against the head top non-observation region of the face 3 inserted in the casing 2, and a jaw rest 10 for restricting the position of the jaw in accordance with the imaging direction such that the front side of the face and the right or left cheek are opposed to the imaging camera.

The head support member 9 includes a vertical position adjusting shaft 12 having a support shaft 11 attached to the lower end for supporting the head top non-observation region of the face 3 at one point is attached to an arm 13 swinging rightward and leftward in the casing 2.
A forward-to-backward position adjusting knob 11a is disposed to the support shaft 11 for adjusting the forward-to-backward position thereof, and a vertical position adjusting knob 12a is disposed to the vertical position adjusting shaft 12 for adjusting the vertical position of the support shaft 11.
The head top non-observation region referred to herein is a region which is not an object for observing the state of skin even when the images thereof are taken and this is, for example, in the vicinity of the boarder of hairs if any, or on the side toward the head top.

Further, in the jaw rest 10, jaw rest portion 10a to 10c, three in all, are formed being arranged at the front side and both right and left sides each with an angle of center of 45° in accordance with the imaging direction for the front side and 45° on the right and the left.
A protrusion 14 abutted against the back of the low jawbone upon resting the jaw is formed to each of the jaw rest portions 10a to 10c.

An example of the constitution of the present invention is as has been described above and the operation thereof is to be described.
At first, the support shaft 11 of the head support member 9 is situated at the front side and the adjusting operation for the forward-to-backward and vertical positions thereof is conducted to situate the head support member 9 to the out side of the non-observation region such that the face is held at the focused focal point of the imaging camera 4.
Since the position does not vary so greatly among individuals, it is not necessary for re-positioning on every imaging once it has been positioned.

Then, when face images at the front side are taken, the face is inserted into the opening 6 in a state where the support shaft 11 situates at the front side, the head top of the face 3 is abutted against the top end of the support shaft 11, the jaw is rested on the jaw rest portion 10b at the center and, when the back of the lower jawbone is abutted against the protrusion 14, since the two vertical points are positioned in a state of directing the face to the front side, images can be taken always under a constant condition.

Further, in a case of taking the face images at 45° on the right, after moving the arm 13 of the head support member 9 to move the support shaft 11 to 45° on the left, the face is inserted into the opening 6 being directed to 45° on the left, the head top side is abutted against the top end of the support shaft 11, the jaw is rested on the jaw rest portion 10a on the left, and the back of the lower jawbone is abutted against the protrusion 14, since the two vertical points are positioned in a state of directing a part of the face 3 at 45° on the right to the front, the face image at 45° on the right can be taken always under a constant condition.

Further, in a case of taking face images at 45° on the left, after moving the arm 13 of the head support member 9 to move the support shaft 11 to 45° on the right, the face is inserted into the opening 6 being directed to 45° on the right, the head top side is abutted against the top end of the support shaft 11, the jaw is rested on the jaw rest portion 10c on the right, and the back of the lower jawbone is abutted against the protrusion 14, since the two vertical points are positioned in a state of directing a part of the face 3 to 45° on the left to the front, face images at 45° on the left can be taken always under a constant condition.

The right illuminated from the illumination light source L to the front side upon imaging is diffused through the light diffusion plate 7, the entire face is illuminated at an uniform brightness in which shining or the like does not cause.
Then, the light reflected at the face 3 and passing through the permeation hole 7a is incident to the imaging camera 4.
Further, since the light illuminated from the illumination light source L to the side of the back and directing to the imaging camera and the light reflected at the back of the light diffusion plate 7 are shielded by the light shielding body 15 located between the illumination light source L and the imaging camera 4, they do not cause weak halation to photographed images which appear whitish but clear images can be obtained.
In addition, in a case of using the reflection mirror for the light shielding body 15, they are reflected toward the light diffusion plate 7 and can be utilized effectively as a illumination light for illuminating the face.

As described above, since the jaw is supported at a position in accordance with the imaging direction of the face 3 and the forehead is supported by the top support member 9, images of the face can be taken at an identical distance and an identical angle relative to the imaging camera 4.
In this case, since the head support member 9 is put against the head top on-observation region such as a portion of hairs which is not necessary as the image data in a case of observing skins, a necessary portion is not in the shade of the support member 9.
Further, when the imaging region is made smaller than the face, since it does not suffer from the effect of the background brightness, it can be imaged at an identical brightness.

### Example 2

Fig. 4 shows another embodiment of the invention. In this embodiment, a head support member 21 is disposed such that it can not be moved and it comprises a vertical position adjusting shaft 24 in which a support shaft 23 having two protrusions 22R, 22L for supporting the head top non-observation region of the face 3 at two right and left points formed at the top end is attached to the lower end. The head support member 21 is fixed instead of the head support member 9 to the ceiling portion of a casing of the face imaging device 1 shown in Fig. 1.
The support shaft 23 is provided with a forward-to-backward position adjusting knob 23a for adjusting the forward-to-backward position thereof, and a vertical position adjusting shaft 24 is provided with a vertical position adjusting knob 24a for adjusting the vertical position of the support shaft 23.
The head support member 21 is fixed instead of the head support member 9 to the ceiling portion of the face imaging device 1 shown in Fig. 1.

Then, upon taking face images at the front side, adjusting operation for the forward-to-backward and vertical position of the support shaft 23 of the head support member 21 is previously conducted to position the head support member 21 to the outside of the non-observation region.
In this state, when the face 3 is inserted into the opening 6, the front head top side of the face 3 (head top side of both eyes) is abutted against the top end of the protrusions 22R, 22L and the jaw is rested on the jaw rest portion 10b at the center to abut the back of the lower jawbone against the protrusion 14, two right and left points on the head top side of the face 3 and the jaw are supported stably at three points and images can be taken always under a constant condition.

Further, upon taking face images at 45° on the right, when the face is inserted in the opening 6 being directed to 45° on the left, the lateral right surface on the head top side of the face (head top side for the right eye and the right ear) is abutted against the top end of the protrusions 22R, 22L, and the jaw is rested on the jaw rest portion 10a on the left to abut the back of the lower jawbone against the protrusion 14, two right and left points at the right lateral surface on the head top side of the face and the jaw are supported stably at 3 points and images can be taken always under the constant condition.

Further, upon taking the face images at 45° on the left, when the face is inserted in the opening 6 being directed to 45° on the right, the lateral left surface on the head top side of the face (head top side for the left eye and the left ear) is abutted against the top end of the protrusions 22R, 22L, and the jaw is rested on the jaw rest portion 10c on the right to abut the back of the lower jawbone against the protrusion 14, two right and left points at the left lateral surface on the head top side of the face jaw are supported stably at 3 points and images can be taken always under the constant condition.

As described above, according to this embodiment, since the face 3 is supported at three points on the head support member 21 and the jaw rest 10, images of the face can be taken at an identical distance and at an identical angle relative to the imaging camera 4.
In this case, since the head support member 21 is put against the head top non-observation region such as the hair portion which is not necessary as the image data upon observation of the skins, necessary portion is not in the shade of the support member 21.
Further, when the imaging region is made smaller than the face, since it does not suffer from the effect of the background brightness, images can be taken at an identical brightness.

In the foregoing explanation, while description has been made to a case of forming a plurality of jaw rest portions 10a to 10c as the jaw rest 10, a single jaw rest portion may also be adapted to slide in an arcuate manner and may be arranged so as to be movable leftwards and rightwards (for example, by 45° leftwards and rightwards).

### Example 3

Fig. 5 shows other embodiment in which those portions in common with Fig. 1 carry same reference numerals for which detailed descriptions are to be omitted.
The face imaging device 31 of this embodiment is adapted such that even when the illuminance/color temperature of the illumination light changes by aging change or replacement of an illumination light source L, they can be maintained at a predetermined constant illuminance/color temperature.
In a case of taking color images by an imaging camera 4, when a color temperature of an illumination light is different, the color changes even when images of an object of an identical color are taken, which gives a more significant effect on the image than the illuminance.
Particularly, for controlling the color temperature, it is necessary to take a white balance on every time and photographed image data have to be put to color compensation based on the balance data.

Then, this embodiment has an illuminance/color temperature variable light source as an illumination light source L, an illuminance sensor S_{L} and a color temperature detection sensor S_{T} for detecting the illuminance and the color temperature of the illumination light, and a control device 32 for variably controlling the illuminance and the color temperature of an illumination light source to predetermined aimed illuminance and aimed color temperature based on the detected color temperature.
It has two fluorescence lamps (light sources) 33H, 33L for individually illuminating lights of different color temperatures as an illumination light source L, in which the color temperature of the fluorescence lamp 33H is set higher than the aimed color temperature CT₀ and the color temperature of the fluorescence lamp 33L is set lower than the aimed color temperature CT₀.
The illuminance sensor S_{B} and the color temperature detection sensor S_{T} are connected to the input of the control device 32, a stabilizer (not illustrated) for each of the fluorescence lamps 33H, 33L is connected to the output so as to control the illuminance/color temperature based on the detection data from each of the sensors S_{B} and S_{T}.

Fig. 6 is a flow chart showing an example of controlling procedures in the control device 32.
At first, when a switch is turned on, control is started. At step STP1, it stands-by till the fluorescence lamps is stabilized after waiting lapse of a predetermined time (10 min) for example by a timer. Then at step STP2, the correlation color temperature CT_{M} of the illumination light source L is measured by the color temperature detection sensor S_{T}, which is compared with the aimed color temperature CT₀ at step STP3.
In a case where the measured value CT_{M} is smaller than an allowable range, it goes to step STP4 and a control signal is outputted to the stabilizer (not illustrated) to control the ratio of the light amount so as to make the fluorescence lamp 33H of higher color temperature brighter and the fluorescence lamp 33H of low color temperature darker. On the other hand, in a case where the measured value CT_{M} is larger than the allowable range, it goes to step STP5, a control signal is outputted to the stabilizer (not illustrated), and the ratio of the light amount is controlled so as to make the fluorescence lamp 33H of higher color temperature darker and the fluorescence lamp 33H of lower color temperature brighter. Then, it returns to step STP3.
Then, in a case where the measured value CT_{M} is within the allowable range, it goes to step STP6.

At step STP6, the illuminance B_{M} of the illumination light source L is measured by the illuminance detection sensor S_{B}, it goes to STP7, and it is compared with an aimed illuminance B₀.
In a case where the measured value B_{M} is lower than an allowable range, it goes to step STP8 and the stabilizer is controlled such that the sum of the light amount increases while maintaining the ratio of light amount for each of the fluorescence lamps 33H and 33L. On the other hand, in a case where the measured value B_{M} is higher than the allowable range, it goes to step STP9 and the stabilizer is controlled such that the sum of the light amount decreases while maintaining ratio of the light amount for each of the fluorescence lamps 33H and 33L, and then it returns to step STP7.
Then, in a case where the measured value CT_{M} is within the allowable range, since it returns to STP2 and continues the processing, the illuminance and the color temperature are kept within the allowable range for the aimed value.

In the foregoing description, while it is explained to a case of controlling both the illuminance and the color temperature, it is not always necessary to control the illuminance of the illumination light source L but it may suffice to control only the color temperature, if a diaphragm of an image pick-up camera is controlled according to illuminance.
Further, a single light source capable of color control may be also used as an illumination light source L, in which it may suffice that the color temperature is aligned with the aimed color temperature by controlling the color of the illumination light.

As has been described above according to the present invention, in a case where images of the face 3 are taken, for example, from three directions at the front, and at 45° on the right and the left, when the face 3 is inserted with the direction being changed from the opening 6 into the casing 2, since the face is held to the focused focal point of the imaging camera 4 by the face holding mechanism 8 comprising the head support member 9 (21) or the jaw rest 10, it can provide an excellent effect capable of taking images of the face 3 from plural directions under an identical imaging condition by placing it into the casing 2.

### Industrial applicability

The present invention can be used in the application of providing image data for informed consent before surgery and confirmation and explanation to a patient of the effect of the treatment after surgery upon conducting laser treatment of illuminating a laser light to a portion to be treated at the surface or subcutaneous portion of skins, for example, in dermatitis, plastic surgery, orthopedic surgery, etc.

### Brief Description of the Drawings

[Fig. 1] is a cross sectional view of a face imaging device according to the invention.
[Fig. 2] is a partially cut away perspective view with an internal mechanism being omitted.
[Fig. 3] is a front elevational outer looking view.
[Fig. 4] is an explanatory view showing a principal portion of another embodiment.
[Fig. 5] is an explanatory view showing other embodiment.
[Fig. 6] is a flow chart showing an example for the control of illuminance/color temperature.

### Description for References

- 1: face imaging device
- 2: casing
- 3: face
- 4: imaging camera
- 5: computer
- 6: opening
- 7: light diffusion plate
- 8: face holding mechanism
- 9: head support member
- 10: jaw rest
- 10a to 10c: jaw rest portion
- 11: support shaft
- 11a: forward to backward position adjusting knob
- 12: vertical position adjusting shaft
- 12a: vertical position adjusting knob
- 13: arm
- 14: protrusion
- 15: light shielding body
- L: illumination light source

## Claims

1. A face imaging device in which an illumination light source for illuminating a visible light to a face is disposed in a casing having an opening formed at the front thereof for inserting the face as an object to be imaged, a light diffusion plate is disposed between the opening and the illumination light source for diffusing a light illuminated from the illumination light source to the front side of the face, and an imaging camera for imaging the face through a permeation hole formed in the light diffusion plate is located at the back thereof, including
a face holding mechanism for holding the face as an object at a focused focal point of the imaging camera by a head support member capable of adjusting the forward-to-backward and vertical positions for restricting the position of a head by abutment against a head top non-observation region of the face inserted in the casing, and a jaw rest for restricting the position of the jaw in accordance with the imaging direction such that the front and the right or left cheeks of the face are faced to the imaging camera, and
a light shielding body located between the illumination light source and the imaging camera for shielding a light incident to the imaging camera without transmitting the permeation hole, among the lights illuminated from the illumination light source.

2. A face imaging device in which an illumination light source for illuminating a illumination light to a face is disposed in a casing having an opening formed at the front thereof for inserting the face as an object to be imaged, a light diffusion plate is disposed between the opening and the illumination light source for diffusing a light illuminated from the illumination light source to the front side of the face, and an imaging camera for imaging the face through a permeation hole formed in the light diffusion plate is located at the back thereof, including
a light shielding body located between the illumination light source and the imaging camera for shielding a light incident to the imaging camera without transmitting the permeation hole, among the lights illuminated from the illumination light source.

3. A face imaging device according to claim 1 or 2, wherein the light shielding body is formed of a reflection mirror for reflecting a light diverged from the illumination light source toward the imaging camera to the diffusion plate.

4. A face imaging device according to claim 1 or 2, wherein a UV-light source for directly illuminating a UV-light to the face inserted into the opening and an auxiliary light source for directly illuminating an auxiliary illumination light for focusing the imaging camera are located out of the imaging area of the imaging camera and at a position not in the shade of the light diffusion plate as viewed from the opening.

5. A face imaging device in which an imaging camera for imaging a face is disposed in a casing an opening formed for inserting a face as an object to be imaged, including
a face holding mechanism for holding the face as an object at a focused focal point of the imaging camera by a head support member capable of adjusting the forward-to-backward and vertical positions for restricting the position of a head by abutment against a head top non-observation region of the face inserted in the casing, and a jaw rest for restricting the position of the jaw in accordance with the imaging direction such that the front and the right or left cheeks of the face are faced to the imaging camera.

6. A face imaging device according to claim 1 or 5, wherein the protrusion abutted against the back of a lower jawbone is formed to the jaw rest.

7. A face imaging device according to claim 1 or 5, wherein the jaw rest comprises a plurality of jaw rest portions arranged in accordance with the imaging direction.

8. A face imaging device according to claim 1 or 5, wherein the jaw rest includes a jaw rest portion disposed so as to be movable rightwards and leftwards in accordance with the imaging direction.

9. A face imaging device according to claim 1 or 5, wherein the head support member supports the head top non-observation region of the face at one point and is disposed so as to be movable rightwards and leftwards in accordance with the imaging direction.

10. A face imaging device according to claim 1 or 5, wherein the head support member supports the head top non-observation region of the face at two right and left points.

11. A face imaging device according to claim 1, including a color temperature variable light source as the illumination light source, a color temperature detection sensor for detecting the color temperature of the illumination light, and a control device for variably controlling the color temperature of the illumination light source to a predetermined aimed color temperature based on the detected color temperature.

12. A face imaging device according to claim 11, wherein two or more kinds of light sources for illuminating lights of different color temperatures are provided as the color temperature variable light sources and the color temperature is controlled by the ratio of the light amount for each of the light sources.

13. A face imaging device according to claim 1 including an illuminance/color temperature variable light source as the illumination light source, a illuminance sensor and a color temperature detection sensor for detecting the illuminance and the color temperature of the illumination light and a control device for variably controlling the illuminance and the color temperature of the illumination light source to aimed illuminance and aimed color temperature based on the detected color temperature.

14. A face imaging device according to claim 13, wherein two or more kinds of light sources for illuminating lights of different color temperatures are provided as the illuminance/color temperature varying light source, the color temperature is controlled by the ratio of the light amount of each of the light sources, and the illuminance is controlled by the sum of the light amount.
